# DEMANDE DE BREVET EUROPEEN

(11) **EP 0 667 352 A1**
(43) Date de publication de la demande: **16.08.1995**
(21) Numéro de dépôt: 95400117.8
(22) Date de dépôt: 20.01.1995
(51) Int. Cl.: C07K 1/36, C07K 14/78, A61K 38/39, A61L 27/00

(54) **Procédé pour l'élimination des prions dans des collagènes et collagènes ainsi obtenus**

(30) Priorité: 24.01.1994 FR 9400716
(71) Demandeur: IMEDEX, F-69630 Chaponost (FR)
(72) Inventeur: Tayot, Jean-Louis, F-69890 La Tour de Salvagny (FR); Tardy, Michel, F-69005 Lyon (FR); Uhlrich, Sylvie, F-69350 la Mulatiere (FR); Chignon, Thierry, F-69005 Lyon (FR); Pouradier-Duteil, Xavier, F-69005 Lyon (FR)
(74) Mandataire: Bernasconi, Jean

(57) **Abrégé**

L'invention concerne l'élimination des prions (ATNC) dans la préparation de collagènes et les collagènes ainsi obtenus.

Le but de l'invention est de fournir un procédé pour la préparation de collagènes, d'origine animale ou humaine, garantissant une élimination complète et fiable des prions, tout en préservant le plus possible la structure et les propriétés du collagène.

Ce but est atteint à l'aide d'un procédé pour la préparation de collagènes selon lequel on soumet le collagène à un traitement alcalin, caractérisé en ce qu'il comprend les étapes consistant à :
- extraire les tissus collagéniques ;
- solubiliser le collagène ;
- éliminer les débris tissulaires ou cellulaires présents dans la solution de collagène obtenue ;
- soumettre le collagène en solution filtrée à un traitement alcalin ;
- isoler le collagène exempt de risques de transmission d'ATNC.

## Description

L'invention concerne l'élimination des risques de contamination des produits biologiques d'extraction par des Agents Transmissibles Non Conventionnels (ATNC), encore appelés "prions". Elle concerne plus particulièrement un procédé garantissant l'élimination des prions pour la préparation de collagènes, destinés notamment à la fabrication de biomatériaux.

Les risques de contamination des produits biologiques d'extraction par des Agents Transmissibles Non Conventionnels (ATNC) font actuellement l'objet d'études approfondies. Ces risques ont été confirmés chez plusieurs espèces animales et chez l'Homme.

En ce qui concerne les espèces animales, la tremblante du mouton existe dans les élevages depuis plusieurs centaines d'années. Depuis 1986, au Royaume-Uni, une épidémie d'Encéphalite Bovine Spongiforme (BSE) touche les bovins et plus de 70 000 cas ont été rapportés à ce jour. L'hypothèse aujourd'hui retenue pour expliquer cette épidémie catastrophique de BSE serait l'utilisation de farines de viande de moutons contaminés, provenant des équarissages britanniques, dans l'alimentation des jeunes veaux. Cette hypothèse semble indiquer la possibilité de passage d'une espèce à une autre, même par voie orale, ce qui évidemment a des conséquences au moins psychologiques et médiatiques pour ce qui concerne les risques de transmission à l'Homme.

Chez l'homme, une maladie dégénérative comparable et mortelle est connue depuis des dizaines d'années. Il s'agit de la maladie de Creutzfeldt-Jakob (CJD), dont la prévalence dans le monde est d'environ 0,6 cas par million d'habitants. La CJD est stable à ce niveau depuis qu'elle est identifiée et recensée ; elle ne semble pas en rapport avec les cas de tremblante du mouton ou d'épidémie de BSE. Cependant, l'incubation très longue de ces maladies, pouvant atteindre plusieurs dizaines d'années, ne facilite pas les études de corrélation ou les observations isolées, et renforce les doutes et l'intérêt des mesures de prévention.

Des cas de transmissions accidentelles de CJD à l'Homme ont été rapportés. La maladie de Kuru, forme de symptomatologie voisine de la CJD, restreinte à certaines tribus anthropophages de Nouvelle Guinée, a disparu depuis que les rites de prélèvements et de consommation des cerveaux d'ancêtres défunts ont été abandonnés, grâce aux découvertes et à l'action du Dr GADJUSEK. Des cas de transmission de CJD ont été rapportés en neurochirurgie chez des malades ayant été au contact d'instruments contaminés, mal stérilisés (BERNOUILLI et coll., 1977 FONCIN et coll., 1980 WILL et coll., 1982), ou ayant reçu des greffes de cornée ou de dure-mère prélevées sur des cadavres (DUFFY et coll., 1974 PRICHARD et coll., 1992 ; MASULO et coll., 1989 ; MIYASHITA et coll., 1991 ; NISBET et coll., 1989 ; POCHIARI et coll., 1992). Des transmissions aux neurochirurgiens ou à leurs collaborateurs ont été décrites également (SCHOENE et coll., 1981 ; MILLER, 1988 ; SITWELL et coll., 1988 ; GORMAN et coll., 1992).

Par ailleurs, l'utilisation d'hormones extraites d'hypophyses humaines et de tissus nerveux associés a entraîné plusieurs dizaines de cas de contamination d'enfants traités pour nanisme, par l'hormone de croissance (POWELL-JACKSON et coll., 1985 ; KOCH et coll., 1985 ; GIBBS et coll., 1985 ; TINTNER et coll., 1986 ; CROXSON et coll., 1988 ; MARZEWSKI et coll., 1988 ; NEW et coll., 1988 ; MACARIO et coll., 1991 ; FRADKIN et coll., 1991 ; BUCHANAN et coll., 1991 ; BROWN et coll., 1992 ; BILLETTE DE VILLEMEUR et coll., 1992) et de deux femmes traitées pour stérilité, par des gonadotrophines (COCHIUS et coll., 1990).

A ce jour, seul le tissu nerveux est reconnu par l'unanimité des experts comme source majeure, sinon exclusive, d'ATNC. De nombreux autres tissus humains ou animaux sont utilisés dans l'industrie biologique et, pour l'instant, aucun tissu non nerveux n'a été à l'origine d'une transmission d'ATNC documentée et confirmée. Des doutes existent sur les risques de transmission d'ATNC à partir d'organes riches en cellules lymphoïdes et une classification des tissus en fonction de ces risques à été proposée par l'O.M.S. : WHO 1991.

Grâce à des modèles animaux particuliers, différentes publications rapportent la présence de prions transmissibles dans le placenta de brebis infectées (PATTISON et coll., 1972 et 1974), ainsi que dans le placenta, le plasma et les lymphocytes d'une patiente atteinte de CJD (TAMAI et coll., 1992), dans les concentrés leucocytaires ou le sang total de patients atteints de CJD (MANUELIDIS et coll., 1985) ou de personnes en bonne santé (MANUELIDIS et coll., 1993). Ces différents travaux et résultats n'ont pas été confirmés par d'autres auteurs (BROWN et coll. 1984) et les critiques émises au sujet des conditions expérimentales, suspectes de contamination de laboratoire, demandent des confirmations préalables à toutes conclusions définitives.

Quelle que soit la réalité de ces risques et en l'absence de diagnostic préalable de ces ATNC, le facteur de sécurité le plus fiable pour l'avenir réside dans la qualité des procédés de purification et/ou d'inactivation utilisés dans la préparation des produits biologiques d'extraction. Aussi, il devient nécessaire, sous la pression des autorités réglementaires et compte tenu des normes de sécurité mises en place, de pouvoir garantir l'efficacité de ces procédés en ce qui concerne leur capacité à éliminer les ATNC. En outre, les exigences de sécurité pour un produit dépendent évidemment des risques associés à ce produit, mais aussi des bénéfices apportés aux patients. C'est ainsi que dans tous les cas où le bénéfice pour le patient n'est pas majeur, ou dans les cas où des produits de substitution aussi efficaces existent, les produits biologiques d'extraction devront fournir le maximum de garanties.

Les collagènes, d'origine humaine ou animale, qui sont utilisés aujourd'hui en chirurgie dans de nombreux biomatériaux, font partie de ces produits biologiques pour lesquels on recherche à garantir l'élimination des prions. Les propriétés des collagènes permettent leur utilisation comme agents hémostatiques, guides de réparation tissulaire, produits de comblement, adhésifs, lentilles cornéennes, tissus reconstitués par des méthodes de réticulation. L'intérêt des collagènes est leur excellente biocompatibilité qui leur permet d'exercer la fonction recherchée, puis de disparaître par résorption en quelques jours, quelques semaines ou quelques mois, en fonction de leur mode de réticulation.

Il est généralement admis que les collagènes animaux sont dépourvus des risques de transmission d'ATNC, notamment pour les raisons suivantes :
- la peau ou les tendons de jeunes veaux qui sont utilisés pour la préparation de collagène bovin (de type I principalement) n'ont jamais été considérés comme vecteurs d'ATNC, y compris lorsqu'ils proviennent d'animaux malades (WHO 1991) ;
- les animaux sources de ces tissus proviennent d'élevages contrôlés, indemnes de BSE, et sont soumis à des contrôles sanitaires rigoureux.

En outre, les tissus animaux utilisés sont quelquefois soumis à des traitements alcalins préalables, destinés à l'élimination des poils de la peau et de certaines impuretés solubles dans ces conditions, en particulier des substances protéiques kératiniques et élastiques (brevet français n° 1 568 829, Nov. 1967). Les auteurs précisent que les substances collagéniques sont relativement intactes après séparation.

Pour certaines préparations, la peau de jeune boeuf est soumise, avant tannage, à un trempage dans une solution aqueuse comprenant de la soude 0,3 à 1,O N avec 10 - 25 % (p/p) de sulfate de sodium et 0,05 - 0,3 M d'un composé aminé à une température de 15 - 25°C ; le temps d'action varie de quelques heures à plusieurs jours (brevet japonais n° 140 582 de 1976, NIPPI Incorporated). Dans ces conditions, les auteurs affirment que des produits destinés à des applications médicales peuvent être préparés à partir du collagène obtenu, de manière à présenter un très faible pouvoir antigénique en prolongeant le traitement alcalin et en favorisant la décomposition des télopeptides.

De même, le brevet américain n° 4 511 653 décrit la préparation de collagènes humains par traitement de tissus placentaires avec de la soude 0,5 M pendant 48 heures à une température inférieure à 10°C. Un des avantages mis en avant par les auteurs est l'élimination des virus tels que l'hépatite B dans ces conditions alcalines.

La demande de brevet français n° 92 00739 décrit un procédé de préparation de collagènes par traitement alcalin de peaux animales par de la soude (ou de la potasse) à une concentration de 1 N pendant 0,5 à 1,5 heures à une température ne dépassant pas 30 à 32°C, avant l'extraction du collagène. Les auteurs semblent étonnés qu'il n'y ait pas de modification de la structure hélicoïdale du collagène ni de sa structure moléculaire. Ils affirment aussi obtenir des fibres de collagène qui présentent un pouvoir hémostatique 1,5 à 2,5 fois supérieur à des fibres de collagène obtenues par un procédé ne se différenciant que par l'absence de l'étape de traitement alcalin.

Il est généralement admis que les traitements alcalins sont efficaces pour inactiver les ATNC. Le traitement par de la soude 1 N pendant 1 heure à 20°C est reconnu aujourd'hui comme l'une des rares solutions possibles pour la décontamination des produits biologiques. Ce traitement est d'ailleurs recommandé par l'O.M.S. (WHO 1991) chaque fois qu'il est possible.

Cependant, l'efficacité de ce traitement soude dépend des conditions expérimentales et des souches d'ATNC (extraits de cerveaux d'animaux infectés) utilisées dans les modèles animaux.

En effet, P. BROWN et coll., (1984) décrivent une réduction d'infectivité de 5,5 log10 DL50 d'une souche de CJD après traitement par de la soude 0,1 N ou 1 N pendant 1 heure. Avec cette souche de CJD, aucune infectivité résiduelle n'est détectable.

P. BROWN et coll. (1986) décrivent également des réductions d'infectivité de plus de 5 log10 DL50 pour une souche de CJD et plus de 6,8 log10 DL50 pour une souche de tremblante de mouton après traitement par de la soude 1 N pendant 1 heure. Aucune infectivité résiduelle n'est détectable dans les deux cas. Une infectivité résiduelle est observée dans le cas d'un traitement par de la soude 0,1 N.

Tandis que DI MARTINO et coll. (1992) décrivent une réduction d'infectivité de 6 log10 DL50 pour une souche de tremblante après traitement par de la soude 1 N pendant 1 heure à température ambiante, avec cependant une infectivité résiduelle détectable dans l'échantillon contaminé, traité à la soude et injecté non dilué.

Face à cet état de la technique, la déposante a voulu tester l'efficacité relative du traitement par NaOH 1 N pendant 1 heure à une température voisine de 20°C en particulier pour des collagènes d'origine placentaire.

Ce traitement a été appliqué à un broyat de tissus placentaires contaminés par un broyat de cerveau de souris infecté par la souche de tremblante de mouton NIH C 506/M3 au sixième passage. L'animal d'expérience était la souris C57B16. Après traitement des tissus par 5 volumes de NaOH 1,2 N pendant une heure, les collagènes ont été précipités par addition d'HCl jusqu'à pH voisin de 3 à +8°C. Le précipité obtenu a été recueilli par centrifugation, puis soumis à plusieurs lavages à température ambiante par un mélange acétone-eau 80:20 v/v et enfin lavage par de l'acétone pure pour obtenir, après séchage sous flux laminaire, une poudre collagénique. Cette poudre collagénique a été digérée par de la collagénase, de manière à obtenir une solution fluide, et injectée en totalité par fractions de 20 µl à des souris par voie intracérébrale au niveau de l'hippocampe droit.

Des essais ont été réalisés en parallèle sur des tissus contaminés non soumis au traitement alcalin servant de témoin positif, ou d'autres essais réalisés à partir de tissus placentaires non contaminés servant de témoin négatif.

Il a été ainsi possible de conclure que le traitement soude 1 N appliqué pendant 1 heure à une température voisine de 20°C ne permet pas d'inactiver complètement la souche de tremblante utilisée. A partir d'un titre infectieux de départ de 10⁸ DL50/g de broyat de cerveau, seule une inactivation d'environ 4 log10 DL50 a été observée.

Contrairement aux résultats antérieurs publiés, plus optimistes, il n'est donc pas évident d'obtenir, à partir de tissus volontairement contaminés au préalable par des ATNC, des préparations de collagène complètement débarassées des risques de présence résiduelle d'ATNC.

Il est certain que ces résultats laissent planer un doute très important sur la possibilité de valider les procédés de purification des collagènes, même lorsqu'une étape de traitement alcalin est présente.

Le but de la présente invention est de fournir un procédé pour la préparation de collagènes, d'origine animale comme d'origine humaine, garantissant une élimination complète et fiable des ATNC.

Le but de l'invention est encore de réaliser une inactivation totale et fiable des ATNC tout en préservant la structure et les propriétés des molécules de collagène solubles.

Un autre but de l'invention est enfin de définir les conditions optimales du traitement alcalin appliqué à des collagènes en solution.

A cette fin, l'invention a pour objet un procédé pour la préparation de collagènes selon lequel on extrait les tissus collagéniques et on solubilise le collagène, le collagène étant soumis à un traitement alcalin, caractérisé en ce que, en vue de l'élimination des ATNC il comprend les étapes consistant à :
- extraire les tissus collagéniques ;
- solubiliser le collagène ;
- éliminer les débris tissulaires ou cellulaires présents dans la solution de collagène obtenue ;
- soumettre le collagène en solution à un traitement alcalin ;
- isoler le collagène exempt de risques de transmission d'ATNC.

L'invention a également pour objet les compositions à base de collagène ou de ses dérivés obtenues par le procédé précité, exemptes de risques de transmission d'ATNC ainsi que les biomatériaux réalisés à partir des collagènes obtenus.

Les inventeurs ont découvert, d'une manière surprenante, que tout risque de contamination résiduelle par des ATNC pouvait être écarté lors de la préparation de collagènes, qu'ils soient d'origine animale ou humaine, d'une part, en éliminant les débris tissulaires ou cellulaires présents avec le collagène préalablement à son traitement alcalin et d'autre part, en réalisant ledit traitement alcalin dans des conditions spécifiques.

Ainsi, selon l'invention, le problème de l'efficacité et de la fiabilité du traitement d'inactivation des ATNC est résolu notamment en procédant, après extraction des tissus collagéniques selon des méthodes classiques, à l'élimination des débris tissulaires ou cellulaires par filtration sur une membrane de porosité inférieure ou égale à 1,2 µ, ou par tout moyen convenable d'élimination des débris, par exemple centrifugation, puis en procédant au traitement alcalin du collagène en solution.

Cette étape de filtration impose, toutefois, de n'utiliser que des collagènes solubilisés au préalable, soit par digestion enzymatique des liaisons covalentes reliant les chaînes de collagène entre elles, soit par coupure alcaline de ces mêmes liaisons.

Il est essentiel de noter ici que les méthodes de traitement alcalin des collagènes, connues jusqu'à ce jour, étaient toujours appliquées sur les tissus solides, puisque leur but était, au mieux, de solubiliser partiellement ces collagènes et d'éliminer certaines impuretés.

En outre, les conditions de traitement à la soude, permettant d'inactiver les ATNC et, parallèlement, de préserver les propriétés de solutions de collagène, n'étaient pas encore connues.

Un objectif important de l'invention est donc d'assurer l'inactivation efficace des ATNC tout en préservant le plus possible la structure et les propriétés du collagène.

Aussi, conformément à l'invention, seul le traitement alcalin, appliqué sur une solution de collagène préalablement solubilisé et filtré, permet d'éliminer toute trace d'ATNC, lorsqu'il y a eu contamination volontaire du tissu collagénique initial.

Le traitement alcalin conforme à l'invention consiste à ajouter, à une solution de collagène, de la soude dont la concentration est comprise entre 0,1 N et 2 N et à laisser agir pendant environ 1 heure sous agitation, à une température de l'ordre de 20°C.

Selon l'invention, on définit avantageusement les conditions de traitement en fonction du type de collagène.

Pour les collagènes de type I ou III en solution et préalablement filtrés à l'état dilué sur membranes de porosité inférieure ou égale à 1,2 micron, les conditions de traitement alcalin sont avantageusement une durée de 60 à 70 minutes, une température de 20°C ±3°C et une concentration en soude comprise entre 0,1 N et 2 N, de préférence 1 N. Si l'on augmente la concentration en soude au-delà de 2 N, ou si l'on augmente le temps de contact au-delà de 2 à 3 heures, le point iso-électrique et la migration électrophorétique des molécules de collagène se modifient nettement. Les propriétés modifiées des collagènes rendent alors plus difficiles certaines applications.

Pour le collagène de type IV en solution et préalablement filtré à l'état dilué sur membranes de porosité inférieure ou égale à 1,2 micron, les conditions de traitement alcalin sont avantageusement une durée de 60 à 70 minutes, une température de 20°C ±3°C et une concentration en soude voisine de 0,1 N. Si l'on augmente le temps de traitement alcalin ou la concentration en soude au-delà de 0,1 N, la viscosité des solutions de collagène IV diminue fortement et ne permet plus l'obtention de gels de viscosité suffisante dans certaines applications. Pour des applications indépendantes de la viscosité, un traitement par la soude 1 N est possible et apporte une garantie formelle supplémentaire de l'élimination des ATNC.

Le collagène inactivé est ensuite précipité et isolé sous la forme désirée telle que par exemple poudre ou gel.

Le collagène est ainsi obtenu selon une méthode de préparation qui peut être officiellement validée auprès des autorités réglementaires de la santé.

Le collagène ainsi obtenu ne présente plus de risque de transmission d'ATNC même en cas de contamination accidentelle des tissus collagéniques. Sa structure hélicoïdale et moléculaire est préservée. Ses propriétés telles que point iso-électrique ou encore viscosité peuvent également être préservées.

Le collagène selon l'invention peut servir à la préparation de compositions de biomatériaux à usage médical ou chirurgical tels que produits injectables, produits hémostatiques, colles biologiques pour la liaison de tissus entre eux ou avec un biomatériau implanté, produits de comblement, produits cicatrisants, etc.

L'invention sera mieux comprise à la lecture des exemples donnés ci-après à titre illustratif et non limitatif.

### EXEMPLES

### I - PREPARATION DE COLLAGENES INTERMEDIAIRES.

### Exemple 1.

Des collagènes humains de type I, III ou IV sont extraits selon les méthodes décrites dans la demande de brevet français n° 85 13004, par digestion du tissu placentaire avec la pepsine, puis séparation et purification des trois types de collagène par des précipitations salines à pH acides et neutre.

### Exemple 2.

Du collagène bovin est préparé à partir de derme ou de tendons de jeunes veaux par le procédé décrit dans la demande de brevet français n° 81 22691 selon lequel le collagène est solubilisé par action de la soude.

Les peaux de veaux provenant d'animaux fraichement abattus sont lavées à l'eau par brassage pendant 1 heure dans une cuve. Le système pileux et le tissu sous-cutané sont séparés du derme à l'aide d'une refendeuse à bande rotative. Le derme récupéré est haché et broyé. Le broyat est lavé par trois bains successifs de tampon phosphate pH 7,8. Entre chaque bain, le broyat est séparé de la solution par centrifugation en continu entre 1000 et 4000 tours/minute. Le résidu est alors rincé par deux bains successifs d'eau permutée et le liquide est séparé du broyat par centrifugation. Ces premiers lavages servent à éliminer des substances non collagéniques. Le tissu est alors placé dans une cuve contenant une solution de soude 1 N à une température voisine de +4°C pendant une période de 1 à 10 jours. Le milieu est ensuite acidifié par l'acide chlorhydrique jusqu'à un pH inférieur à 3, en ajoutant du chlorure de sodium de manière à atteindre une concentration de 100 g/l. Le collagène précipité est dialysé contre de l'eau permutée.

Ce produit ayant subi une attaque alcaline prolongée n'est pas préféré lorsque les propriétés électriques de la molécule de collagène doivent être aussi proches que possible de l'état initial, et compte tenu de l'hétérogénéité des molécules obtenues (certaines étant des monomères, d'autres des polymères, d'autres des agrégats insolubles, plus ou moins modifiés en fonction des conditions d'action plus ou moins importantes de la soude au niveau tissulaire).

### Exemple 3.

Du collagène bovin est préparé à partir de dermes de jeunes veaux lavés comme précédemment et soumis à une digestion par la pepsine en tampon acide citrique 0,05 M pH 2,4. La dose de pepsine est d'environ 40 g par kg de tissu collagénique. La durée de la digestion est de 60 heures environ à 17°C ±2°C.

Comme dans les exemples précédents, le collagène solubilisé est ensuite purifié par précipitation saline à pH acide et à pH neutre. Les séparations de précipité s'effectuent par centrifugation en continu.

### II - INACTIVATION DE COLLAGENES

### Exemple 4.

Les précipités obtenus selon les exemples 1,2 ou 3, de collagène humain ou bovin de type I ou III sont solubilisés en acide citrique 0,05 M à une concentration de 1 à 2 g/l. Après dissolution pendant au moins 8 heures, la solution obtenue est centrifugée pour éliminer les agrégats insolubles puis filtrée sur membranes jusqu'à une porosité d'environ 1,2 micron (une porosité de 0,45 micron est préférable chaque fois qu'une stérilité bactériologique est recherchée).

La solution acide filtrée est additionnée de chlorure de sodium à 41 g/l (il faut augmenter la teneur en chlorure de sodium à 100 g/l pour des collagènes prétraités longuement à la soude). Après repos pendant une nuit à une température d'environ 10°C, le précipité de collagène est récupéré par centrifugation en continu. Le précipité est redissout avec de l'acide chlorhydrique 0,01 N à une concentration d'environ 8 g/l. La solution limpide obtenue est neutralisée à pH 7 par addition de soude 2 N.

On ajoute 1 volume de soude 2 N à 1 volume de la solution collagénique précédente et le mélange est maintenu en agitation pendant 60 à 70 minutes à 20°C ±3°C, ce qui donne une concentration finale en soude de 1 N.

Le mélange est ensuite dilué 5 fois avec de l'eau déminéralisée et aussitôt neutralisé à pH 7,5 avec de l'acide citrique 1 M. Le collagène est précipité par ajustement à 100 g/l de NaCl à 20°C à pH neutre, ou par ajustement à 41 g/l de NaCl à pH 2,8 et à 10°C.

Le précipité de collagène est lavé à l'acétone pour obtenir une poudre permettant la préparation de divers biomatériaux connus selon les méthodes classiques.

### Exemple 5.

Pour le collagène de type IV, on procède comme à l'exemple 4 à la seule différence que la concentration finale en soude est réduite à une valeur de 0,1 N.

### III - TEST D'EFFICACITE DU PROCEDE SELON L'INVENTION.

Pour tester l'efficacité du procédé, on peut préparer des collagènes à partir de dermes ou de placentas préalablement mélangés avec un poids de 1/10° de cerveau de souris infectée par la souche de tremblante NIH C 506/M3 au sixième passage et titrant environ 10⁸ DL50/g de broyat de cerveau.

Les poudres acétoniques de collagène obtenues selon les exemples précédents sont digérées à la collagénase de manière à obtenir une solution fluide non toxique pour le cerveau, et injectées en totalité par fractions de 20 µl à des souris par voie intracérébrale, au niveau de l'hippocampe droit.

On vérifiera l'absence de symptômes d'encéphalite aigüe dégénérative après un recul de 18 mois, alors que les souris témoins sont toutes mortes.

### BIBLIOGRAPHIE

- C. BERNOULLI et al., (1977) THE LANCET, i, 478-479, "Danger of accidental person-to-person transmission of Creutzfeldt-Jakob disease by surgery",
- T. BILLETTE DE VILLEMEURE et al., (1992) REV. NEUROL., 148, 5, 328-334, "Maladie de Creutzfeldt-Jakob chez quatre enfants traités par l'hormone de croissance",
- P. BROWN et al., (1984) ANN. NEUROL., 16, 295, "Creutzfeldt-Jakob disease of long duration. Clinico-pathological characteristics. Transmissibility and differential diagnosis",
- P. BROWN et al., (1984) THE NEW ENGLAND JOURNAL OF MEDECINE, 310, 11, 727, "Sodium hydroxyde decontamination of Creutzfeldt-Jakob disease virus",
- P. BROWN et al., (1986) THE JOURNAL OF INFECTIOUS DISEASE, 153, 6, 1145-1148, "Newer data on the inactivation of Scrapie or Creutzfeldt-Jakob disease virus in brain tissue",
- P. BROWN et al., (1992) THE LANCET, 340, 24-27, "Friendly fire in medecine : hormones, homografts, and Creutzfeldt-Jakob disease"
- C.R. BUCHANAN et al., (1991) BR. MED. J., 302, 824-828, "Mortality, neoplasia, and Creutzfeldt-Jakob disease in patients treated with human pituitary growth hormone in the United Kingdom",
- J.I. COCHIUS et al., (1990) AUST. N. Z. J. MED., 20, 592-593, "Creutzfeldt-Jakob disease in a recipient of human pituitary-derived gonadotropin ?"
- M. CROXSON et al., (1988) NEUROLOGY, 38, 1128-1130, "A new case of Creutzfeldt-Jakob disease associated with human growth hormone therapy in New Zealand",
- A. DI MARTINO et al., (1992) ARCHIVE OF VIROLOGY 124, 111-121, "Purification of non infectious ganglioside preparations from Scrapie-infected brain tissues",
- P. DUFFY et al., (1974) N. ENGL. J. MED., 290, 692-693, "Possible person-to-person transmission of Creutzfeldt-Jakob disease",
- J.F. FONCIN et al., (1980) REV. NEUROL., 136, 280, "Transmission iatrogène interhumaine possible de Creutzfeldt-Jakob avec atteinte des grains du cervelet",
- J.E. FRADKIN et al., (1991) JAMA, 265, 880-884, "Creutzfeldt-Jakob disease in pituitary growth hormone recipients in United States",
- D.J. GIBBS et al., (1985) N. ENGL. J. MED., 313, 734-738, "Clinical and pathological features and laboratory confirmation of Creutzfeldt-Jakob disease in a recipient of pituitary-derived human growth hormone?",
- D.G. GORMAN et al., (1992) NEUROLOGY, 42, 463, "Creutzfeldt-Jakob disease in a pathologist",
- T.K. KOCH et al., (1985) N. ENGL. J. MED., 313, 731-733, "Creutzfeldt-Jakob disease in a young adult with idiotypic hypopituitarism. Possible relation to the administration of cadaveric human growth hormone",
- M.E. MACARIO et al., (1991) BR. MED. J., 302, 1149, "Pituitary growth hormone and Creutzfeldt-Jakob disease",
- E.E. MANUELIDIS et al., (1985) THE LANCET, Octobre 19, 896-897, "Transmission to animals of Creutzfeldt-Jakob Disease from human blood",
- E.E. MANUELIDIS et al., (1993) PRAC. NATL. ACAD. SCI., 90, 896-897, "A transmissible Creutzfeldt-Jakob disease-like agent is prevalent in the human population",
- C. MASULLO et al., (1989) J. NEUROSURG. 71, 954-955, "Transmission of Creutzfeldt-Jakob disease by dural cadaveric graft",
- D.J. MARZEWSKI et al.,(1988) NEUROLOGY, 38, 1131-1133, "Creutzfeldt-Jakob disease following pituitary-derived human growth hormone therapy : a new american case",
- D.C. MILLER, (1988) N. ENGL. J. MED., 318, 853-857, "Creutzfeldt-Jakob disease in histopathologist technicians",
- K. MIYASHITA et al., (1991) NEUROLOGY, 41, 940-941, "Creutzfeldt Jakob disease in a patient with a cadaveric dural graft",
- M.I. NEW et al., (1988) NEUROLOGY, 38, 1133-1134, "Preclinical Creutzfeldt-Jakob disease discovered at autopsy in a human growth hormone recipient",
- T.J. NISBET et al., (1989) JAMA, 261, 11-18, "Creutzfeldt-Jakob disease in a second patient who received a cadaveric dura mater graft",
- I.H. PATTISON et al., (1972) THE VETERINARY RECORD, 90, 465-468, "Spread of Scrapie to sheep and goats by oral dosing with foetal membranes from Scrapie-affected sheep",
- I.H. PATTISON et al., (1974) BR. VET. J., 130, Ixv, "Further observations on the production of scrapie in sheep by oral dosing with foetal membranes from scrapie-affected sheep",
- M. POCCHIARI et al., (1992) THE LANCET, 340, 614-615, "Creutzfeldt Jakob disease after non commercial dura mater graft",
- J. POWELL-JACKSON et al., (1985) THE LANCET, ii, 244-246, "Creutzfeldt-Jakob disease after administration of human growth hormone".
- J. PRICHARD et al., (1992) JAMA, Feb. 27, 1036, "Dementia in dura mater graft patient",
- C. SCHOENE, et al., (1981) ARCH. NEUROL., 38, 473-477, "Transmissible spongiform encephalopathy (Creutzfeldt-Jakob disease). Atypical clinical and pathological findings",
- L. SITWELL et al., (1988) N. ENGL. J. MED., 318, 854 "Creutzfeldt-Jakob disease in histopathology technicians",
- Y. TAMAI et al., (1992) THE NEW ENGLAND JOURNAL OF MEDICINE, 327, 9, 649, "Demonstration of the transmissible agent in tissue from a pregnant woman with Creutzfeldt-Jakob disease",
- R. TINTNER et al., (1986) NEUROLOGY, 36, 932-936, "Neuropathologic verification of Creutzfeldt-Jakob disease in the exhumed American recipient of human pituitary growth hormone ; epidemiologic and pathogenic implications ?",
- R.G. WILL et al., (1982) J. NEUROL, NEUROSURG. PSYCHIAT., 45, 235-238, "Evidence for case-to-case transmission of Creutzfeldt-Jakob disease",
- WHO/CDS/VPH/92.104, 12-14 Novembre 1991 "Report of a WHO consultation on public health issues related to animal an human spongiform encephalopathies", Genève.

## Revendications

1. Procédé pour la préparation de collagènes dans lequel on extrait les tissus collagéniques et on solubilise le collagène, le collagène étant soumis à un traitement alcalin,
caractérisé en ce que, en vue de l'élimination des ATNC, il comprend les étapes consistant à :
- éliminer les débris tissulaires ou cellulaires présents dans la solution de collagène obtenue ;
- soumettre le collagène en solution à un traitement alcalin ;
- isoler le collagène exempt de risques de transmission d'ATNC.

2. Procédé selon la revendication 1, caractérisé en ce que les tissus collagéniques sont d'origine animale ou humaine.

3. Procédé selon l'une quelconque des revendications 1 et 2, caractérisé en ce que l'on solubilise le collagène par digestion enzymatique.

4. Procédé selon l'une quelconque des revendications 1 et 2, caractérisé en ce que l'on solubilise le collagène par traitement alcalin prolongé des tissus collagéniques.

5. Procédé selon l'une quelconque des revendications précédentes, caractérisé en ce que
pour éliminer les débris tissulaires ou cellulaires, on filtre la solution de collagène sur une membrane de porosité inférieure ou égale à 1,2 micron.

6. Procédé selon la revendication 5, caractérisé en ce que la filtration est réalisée sur une membrane de porosité d'environ 0,45 micron.

7. Procédé selon l'une quelconque des revendications précédentes, caractérisé en ce que l'on soumet le collagène en solution débarassé des débris tissulaires ou cellulaires à l'action de la soude à une concentration comprise entre 0,1 N et 2 N pendant environ 1 heure à une température de l'ordre de 20°C.

8. Procédé selon la revendication 7, caractérisé en ce que pour du collagène de type I ou III, on utilise de la soude de préférence à une concentration d'environ 1 N.

9. Procédé selon la revendication 7, caractérisé en ce que pour du collagène de type IV, on utilise de la soude à une concentration d'environ 0,1 N.

10. Collagène, sous forme de poudre ou de gel, obtenu par le procédé selon l'une quelconque des revendications 1 à 9.

11. Biomatériau réalisé à partir de collagène obtenu par le procédé selon l'une quelconque des revendications 1 à 9.
